# FASCICULE DE BREVET EUROPEEN

(11) **EP 3 549 559 B1**
(45) Date de publication et mention de la délivrance du brevet: **16.06.2021**
(21) Numéro de dépôt: 19305436.8
(22) Date de dépôt: 02.04.2019
(51) Int. Cl.: A61F 5/01

(54) **ORTHÈSE PIED ET JAMBE JUSQU'AU BAS DU GENOU**
ORTHESE FÜR FUSS UND BEIN BIS ZUM KNIE
BRACE FOR FOOT AND LEG UP TO BELOW THE KNEE

(30) Priorité: 05.04.2018 FR 1852981
(43) Date de publication de la demande: 09.10.2019
(73) Titulaire: Espace Ortho Scoliose SAS, 75004 Paris (FR)
(72) Inventeur: KAROUBI, David, 75012 PARIS (FR)
(74) Mandataire: Plasseraud IP

(56) Documents cités:
- WO-A1-2014/093851
- WO-A1-2016/011493
- WO-A2-2006/061603
- DE-A1-102011 121 301
- US-A- 5 472 414
- US-A1- 2008 208 094
- US-B1- 8 007 454

## Description

### Domaine technique

La présente invention concerne le domaine technique des dispositifs orthopédiques et notamment des orthèses de jambe. Plus particulièrement, la présente invention concerne le domaine des orthèses de jambe arrivant sous le genou.

### État de la technique

Il existe dans l'état de la technique des dispositifs orthopédiques adaptés aux personnes souffrant du syndrome du pied tombant. Ce syndrome provoque une anomalie de la marche due notamment à la faiblesse, l'irritation ou une atteinte du nerf fibulaire commun incluant le nerf sciatique, ou à la paralysie des muscles de la partie antérieure de la jambe. Ce syndrome se traduit par l'incapacité ou la capacité réduite de relever les orteils ou le pied à partir de la cheville (dorsiflexion). Le pied tombant peut être temporaire ou permanent en fonction de la gravité de la faiblesse musculaire ou de la paralysie et peut affecter l'un ou les deux pieds.

Les causes du pied tombant comprennent une atteinte nerveuse, un traumatisme musculaire ou de la moelle épinière, une anatomie anormale. Le pied tombant peut aussi avoir comme origine des toxines ou maladies conduisant à une perte de la fonction des voies neurales motrices et sensorielles, conduisant par exemple à une paralysie due à un dysfonctionnement neurologique. Les maladies pouvant occasionner le pied tombant sont par exemple : un accident vasculaire cérébral, la sclérose latérale amyotrophique, la sclérose en plaque, la dystrophie musculaire, la poliomyélite, la maladie de Charcot-Marie-Tooth, l'infirmité motrice cérébrale, la paraplégie spastique familiale, le syndrome de Guillain-Barré, la myopathie distale de Welander et l'ataxie de Friedreich.

Les dispositifs orthopédiques actuels pour traiter le pied tombant sont généralement des dispositifs fabriqués dans des matériaux rigides.

Il existe par exemple une orthèse comprenant une gouttière avec une partie jambière et une partie pédieuse. La partie jambière et la partie pédieuse pouvant avantageusement être réalisées en une seule et même pièce faite d'un seul et même bloc de matière. La gouttière est moulée de manière à épouser au mieux les formes de la jambe et du pied du patient traité. Cette orthèse comprend en outre deux attaches : une première attache disposée sur la partie jambière pour une attache au contact du tibia ; et une deuxième attache disposée à la jonction entre la partie jambière et la partie pédieuse, c'est-à-dire approximativement au niveau de la cheville, pour une attache au contact du dos du pied. Ces attaches sont fixées par rivets sur la coque.

Cette orthèse, bien que fournissant un maintien de la jambe et du pied approprié, présente des inconvénients.

Un premier inconvénient est l'encombrement de l'orthèse. En effet, les systèmes de fixation des attaches sur la coque augmentent l'encombrement de l'orthèse. Ainsi, le patient ne peut porter des chaussures disponibles dans le commerce, sauf à prendre une pointure trop grande pour lui, et doit se faire faire des chaussures orthopédiques adaptées.

Quand bien même l'encombrement de l'orthèse n'était pas un problème, la rigidité de la partie pédieuse empêche au porteur d'insérer son pied dans des chaussures disponibles dans le commerce.

Un autre inconvénient est l'inconfort du port de la prothèse. En effet, les parties anatomiques saillantes sont au contact d'un matériau rigide, ainsi une douleur à ces endroits peut être ressentie par le porteur.

Afin d'améliorer le confort, des coussinets peuvent être prévus notamment au niveau des malléoles, ce qui nécessite l'ajout de rivets pour leur fixation empirant ainsi l'encombrement de l'orthèse.

Par ailleurs, ce type d'orthèse ne permet pas de lutter contre la spasticité qui est une exagération du réflexe ostéotendineux consistant en un étirement rapide d'un muscle qui entraine trop facilement sa contraction réflexe qui dure un certain temps. Au contraire, les orthèses rigides amplifient ce trouble.

WO 2006/061603 A2 décrit une orthèse pour le traitement du pied tombant. US 8007454 B1, WO 2016/011493 A1, US 2008/208094 A1, DE 10 2011 121301 A1, US 5472414 A et WO 2014/093851 A1 décrivent des attelles.

### Présentation de l'invention

Un des objectifs de la présente invention est de pallier au moins un des inconvénients de l'état de la technique décrit ci-dessus.

Pour cela, la présente invention propose une orthèse de jambe comprenant une coque souple et élastique avec une partie jambière et une partie pédieuse s'étendant à partir de la partie jambière, caractérisée en ce que la partie jambière présente une forme tubulaire et en ce que la partie jambière et la partie pédieuse sont réalisées en une seule et même pièce faite d'un seul et même bloc de matière.

La forme tubulaire de la partie jambière permet l'utilisation d'un matériau souple pour fabriquer la coque. L'utilisation d'un tel matériau souple n'est pas évidente. En effet, dans le domaine des orthèses de jambe notamment pour le traitement du pied tombant, existe un impératif: celui de procurer un maintien à la jambe et au pied. C'est pourquoi les orthèse existantes emploient des matériaux rigides. Afin de procurer une rigidité suffisante tout en utilisant un matériau souple, les inventeurs ont eu l'idée de prendre en compte la présence *in fine* de la jambe, dans la conception du dispositif. Ainsi, la rigidité est apportée par la forme tubulaire de la partie jambière qui, une fois remplie par la jambe du porteur, est suffisamment rigide pour soutenir la marche du porteur.

Par ailleurs, la combinaison de la forme tubulaire permettant l'utilisation d'un matériau souple, d'autres systèmes de fixation des attaches moins encombrants peuvent être utilisés. En outre, l'encombrement réduit et/ou la flexibilité de la partie pédieuse permet l'insertion du pied dans une chaussure disponible dans le commerce.

En outre, contrairement aux orthèses rigides, cette orthèse souple permet de lutter contre la spasticité et n'amplifie pas ce trouble.

D'autres caractéristiques optionnelles et non limitatives sont les suivantes.

La seule pièce peut être en un matériau de dureté de 65 à 100 Shore A.

La seule pièce est en un matériau d'élasticité de 15 à 25 N/mm².

La forme tubulaire de la partie jambière peut être obtenue par jointure de deux bords de la coque souple. Auquel cas, l'orthèse peut comprendre en outre un système d'attache apte à maintenir les deux bords sensiblement droits joints. Le système d'attache peut comprendre une attache prévue sur la partie jambière et/ou une attache prévue sur la jonction entre la partie jambière et la partie pédieuse.

La partie jambière peut présenter des orifices de ventilation.

La partie pédieuse peut présenter une partie talon avec un orifice d'évacuation.

La partie pédieuse peut comprendre une partie plantaire de soutien du pied.

La partie pédieuse peut comprendre une ouverture avant de dégagement des orteils. L'orthèse peut comprendre en outre un raidisseur pour l'arrière pied.

### Dessins

D'autres objectifs, caractéristiques et avantages apparaitront à la lecture de la description qui suit donnée à titre illustratif et non limitatif en référence aux dessins, parmi lesquels :
- la figure 1 est une vue de trois-quarts à partir du côté gauche d'un exemple d'orthèse (pied gauche) selon l'invention ;
- la figure 2 est une vue de trois-quarts à partir du côté droit de l'exemple d'orthèse de la figure 1 ;
- la figure 3 est une vue de trois-quarts à partir de la partie talon de l'exemple d'orthèse de la figure 1 ;
- la figure 4 est une vue de côté (côté gauche) de l'orthèse de la figure 1 avec un raidisseur en place ;
- la figure 5 la même vue que la figure 4 mais avec le raidisseur et la coque séparés l'un de l'autre ;
- la figure 6 montre une section transversale droite de la partie jambière dans le cas où les bords de celle-ci se situent à distance l'un de l'autre ;
- la figure 7 montre une section transversale droite de la partie jambière dans le cas où les bords de celle-ci se chevauchent.

### Description

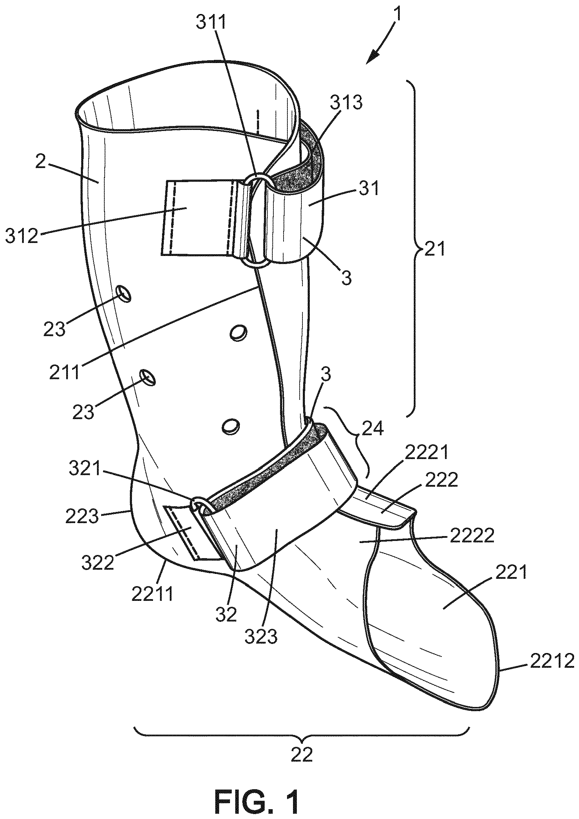

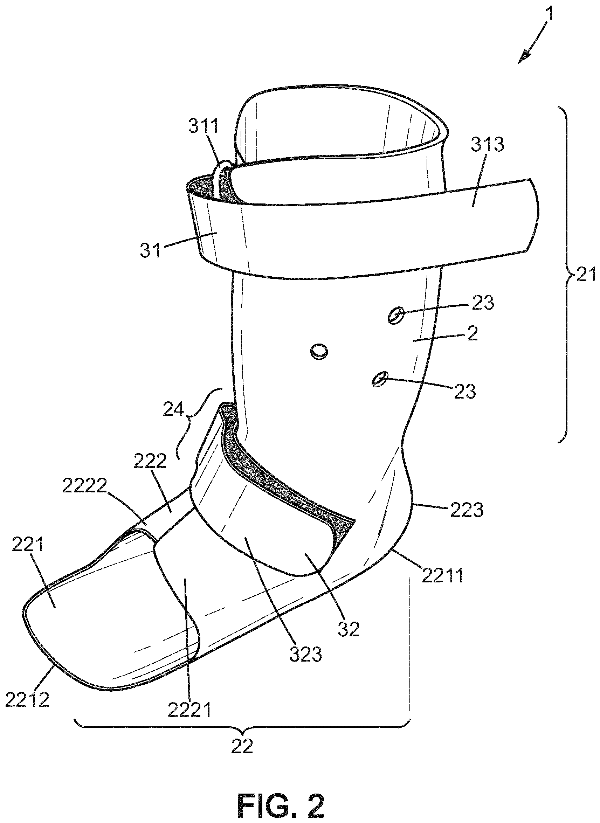

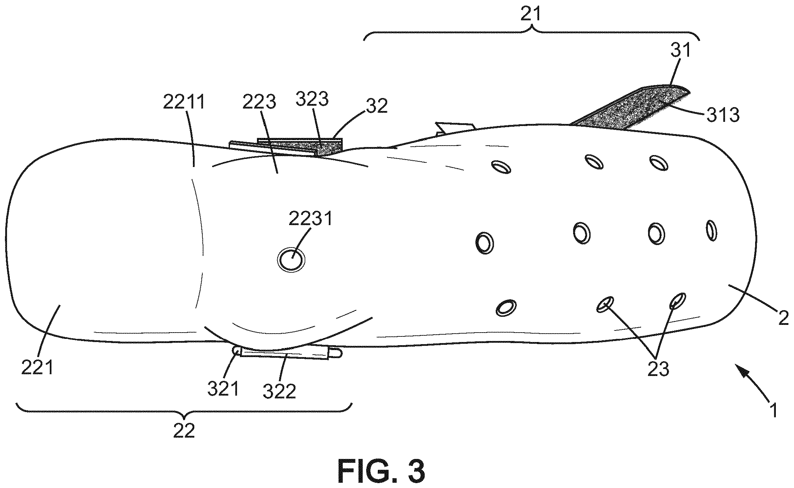

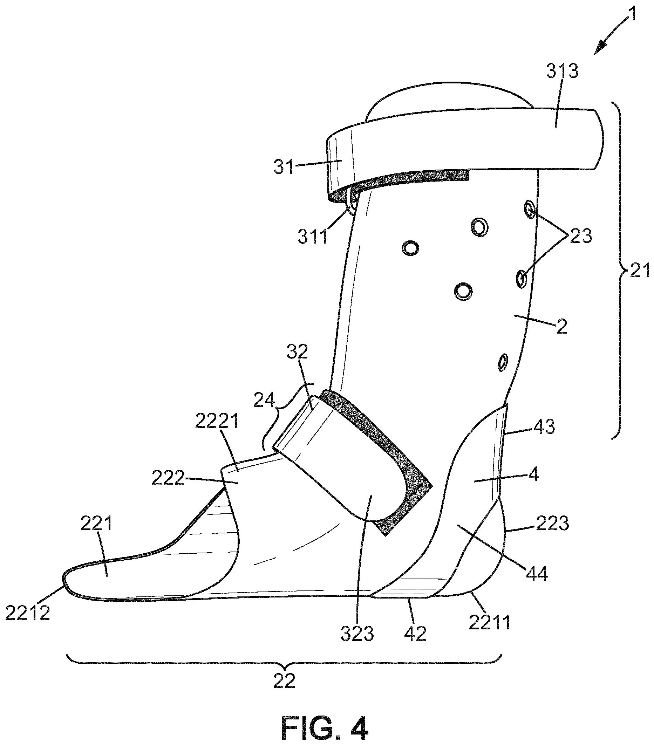

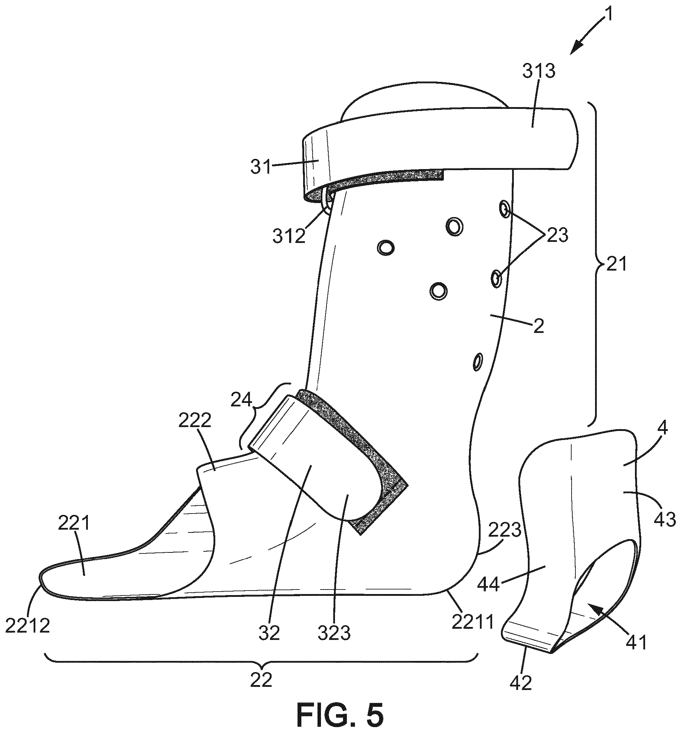

Un exemple d'orthèse selon la présente invention est ci-après décrit en référence aux figures 1 à 7.

L'orthèse 1 est une orthèse de jambe destinée à couvrir la jambe d'un patient du pied jusqu'en dessous des genoux. Cette orthèse permet notamment le traitement du pied tombant. Contrairement aux orthèses à cet effet de l'état de la technique, la présente orthèse est réalisée dans un matériau souple et élastique, avec un module d'élasticité compris entre 15 et 25 N/mm2. On entend par matériau souple un matériau pouvant être facilement déformé par une personne à l'aide de ses mains, sans utiliser d'outil. Notamment un tel matériau présente typiquement une dureté comprise entre 65 à 100 Shore A. Le matériau peut présenter avantageusement une dureté comprise entre 70 et 90 Shore A, entre 75 et 85 Shore A, de préférence environ 82 Shore A. Le matériau peut présenter avantageusement un module d'élasticité comprise entre 17 et 23 N/mm², entre 18 et 22 N/mm², de préférence environ 20 N/mm².

L'orthèse **1** de jambe comprend une coque **2** souple et élastique. La coque **2** présente préférablement une épaisseur comprise entre 2 mm et 5 mm.

La coque **2** est préférentiellement en un matériau à base d'éthylène-acétate de vinyle. Alternativement, le matériau est à base de polyéthylène basse densité. Alternativement, le matériau est à base d'un mélange d'éthylène-acétate de vinyle et de polyéthylène basse densité.

La coque **2** est notamment fabriquée par moulage sur le pied du patient.

La coque **2** comprend une partie jambière **21** et une partie pédieuse **22** s'étendant à partir de la partie jambière **21.** La partie jambière **21** et la partie pédieuse **22** sont réalisées en une seule et même pièce faite d'un seul et même bloc de matière.

La partie jambière **21** présente une forme tubulaire et est destinée à enserrer la jambe du patient. La forme tubulaire de la partir jambière peut être obtenue par l'enroulement d'une feuille de matériau souple et élastique. Le caractère élastique du matériau conduisant à revenir à l'état enroulé hors contrainte lorsque l'on tente de dérouler la feuille. Les bords **211,** lors de l'enroulement, se rapprochent l'un de l'autre de préférence sur le tibia, c'est-à-dire sur la partie avant de l'orthèse. Ces bords **211** peuvent dans l'état enroulé hors contraint se situer à distance l'un de l'autre, en jointure l'un à l'autre ou se chevaucher (comme représenté sur les figures).

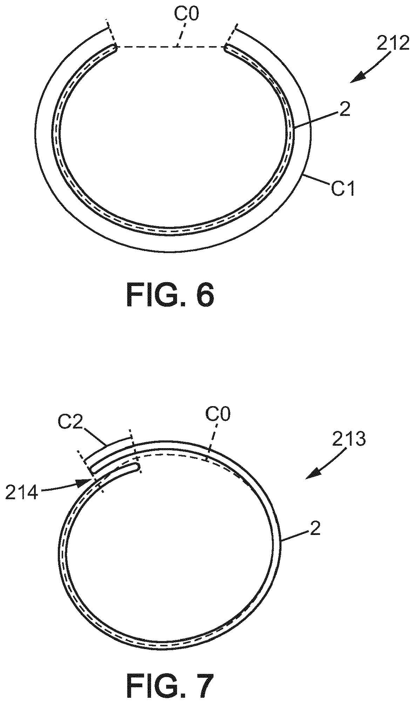

Dans le cas où ils se situent à distance l'un de l'autre, si l'on regarde une section **212** transversale droite de la partie jambière **21** (c'est-à-dire sensiblement perpendiculaire à l'axe longitudinal moyen du tube, voir figure 6), et si l'on prolonge dans la section transversale la courbe formée par le matériau de celle-ci de manière à obtenir un contour fermé, la courbe **C1** formée par le matériau représente au moins 75 % du contour fermé **C0,** de préférence au moins 80 %, 85 %, 90 %, 95 %. Il convient de préciser que le prolongement dont il est question ici est un prolongement respectant la courbure de la courbe formée par le matériau aux extrémités correspondant aux bords.

Le chevauchement des bords conduit à une double épaisseur **214** à cet endroit. Ainsi il est préférable dans ce cas que le chevauchement **C2** représente au plus 15 % du contour fermé **C0,** de préférence au plus 10 %, 5 %. Le contour fermé **C0** est le contour formé par le matériau de la partie jambière lorsqu'une section **213** transversale droit de celle-ci est prise en compte (voir figure 7).

Des orifices d'aération **23** peuvent être prévues sur la partie jambière **21,** ces orifices d'aération **23** étant répartis de préférence suivant un maillage régulier, par exemple triangulaire. De préférence, lorsque les bords de la partie jambière **21** se chevauchent, le chevauchement est exempt d'orifices d'aération.

La partie pédieuse **22** prolonge la partie jambière **21** pour servir d'appui au pied et le recouvrir au moins partiellement. La partie pédieuse **22** comprend notamment une partie plantaire **221** de soutien du pied. Cette partie plantaire **221** s'étend notamment entre le talon **2211** jusqu'au bout des orteils **2212.**

La partie pédieuse **22** comprend typiquement une partie dorsale **222** recouvrant au moins partiellement le dos du pied. De préférence, la partie dorsale **222** présente des dimensions pour ne recouvrir qu'une partie du dos du pied de manière à laisser les orteils exposées. Lorsque la partie jambière **21** est formée à partir d'une feuille de matière enroulée, la partie dorsale **222** comprend alors deux feuillets **2221, 2222,** chacun prolongeant la partie pédieuse **21** au niveau d'un bord **211** correspondant. Ainsi, les bords des feuillets peuvent dans l'état enroulé hors contrainte se situer à distance l'un de l'autre, en jointure l'un à l'autre ou se chevaucher avec les mêmes options que pour les bords de la partie jambière en ce qui concerne les pourcentages de rapprochement et/ou de recouvrement.

La partie pédieuse **22** comprend typiquement une partie talon **223** recouvrant le talon du porteur. Cette partie talon **223** est de préférence munie d'un orifice d'évacuation **2231** de la sueur pouvant s'accumuler à l'intérieur de la partie pédieuse **22.** De préférence, l'orifice d'évacuation **2231** se situe sur la portion arrière de la partie talon **223** afin de permettre l'évacuation de la sueur par simple inclinaison du pied vers le haut sans que l'orifice d'évacuation **2231** ne se fasse trop sentir par le porteur.

L'orthèse **1** peut comprendre en outre un système d'attache **3** apte à maintenir la formue tubulaire de la partie jambière **21** lorsque celle-ci est formée d'une feuille de matière. Le système d'attache **3** n'est pas nécessaire pour obtenir la forme tubulaire, puisque la partie jambière **21** est élastique et que hors contrainte, c'est la forme tubulaire qu'on obtient. Le système d'attache **3** sert surtout au maintien de cette forme tubulaire lors que l'utilisation de l'orthèse **1** par le porteur. En effet, lors de la marche, de la course, du saut ou toute autre activité ou le pied est sollicité, des forces peuvent s'exercer sur la partie jambière **21** de manière à écarter les bords **211** de celle-ci.

De préférence, le système d'attache **3** comprend une attache **31** prévue sur la partie jambière **21.** En outre ou alternativement, le système d'attache **3** peut comprendre une attache **32** prévue sur la jonction **24** entre la partie jambière **21** et la partie pédieuse **22.** Le type d'attache à utiliser n'est pas limité et peut comprendre les systèmes à crochets et boucles textiles (plus connus sous le terme de « Velcro® » ou « scratch »), les systèmes à boutons pression, des systèmes à boucle et sangle, etc. ; les systèmes à crochets et boucles textiles étant préférés pour leur simplicité d'utilisation. Dans le cas d'attaches **31, 32** de type Velcro®, chacune des attaches comprend une boucle **311, 321** liée à un ruban de fixation **312, 322** fixé à la coque par couture, et une bande textile **313, 323** comprenant dans son revers deux portions juxtaposées, une première portion comprenant des crochets textiles et une deuxième portion comprenant des boucles textiles.

Compte tenu du caractère souple de la coque **2,** les attaches **3** peuvent être fixées sur la coque par couture.

L'orthèse **1** peut en outre comprendre un raidisseur **4,** notamment pour l'arrière pied. Le raidisseur **4** présente typiquement une forme identique à la partie de l'orthèse **1** correspondant à l'arrière pied, c'est-à-dire autour de la partie talon **223** de la partie pédieuse **22** avec toutefois un orifice **41** afin de laisser passer la partie talon **223** une fois le raidisseur **4** et la coque **2** assemblés. Le raidisseur **4** comprend une bande plantaire **42** et une plaque arrière **43** reliées entre elles par deux portions latérales **44** de sorte qu'en position debout de l'utilisateur portant l'orthèse **1,** la bande plantaire **42** s'étend sensiblement horizontalement et est disposée plus en avant par rapport à la plaque arrière et au contact avec l'arrière de la partie plantaire **221** de la partie pédieuse **22.** La plaque arrière **43** s'étend sensiblement verticalement et les portions latérales **44** sous forme de bandes s'étendent en biais entre la bande plantaire **42** et la plaque arrière **43.**

## Revendications

1. Orthèse (1) de jambe contre la spasticité comprenant une coque (2) souple et élastique avec une partie jambière (21) et une partie pédieuse (22) s'étendant à partir de la partie jambière, **caractérisée en ce que** la partie jambière présente une forme tubulaire et **en ce que** la partie jambière et la partie pédieuse sont réalisées en une seule et même pièce faite d'un seul et même bloc de matière, dans laquelle la seule pièce est en un matériau d'élasticité de 15 à 25 N/mm².

2. Orthèse de jambe selon la revendication 1, dans laquelle la seule pièce est en un matériau de dureté de 65 à 100 Shore A.

3. Orthèse de jambe selon la revendication 1 ou la revendication 2, dans laquelle la forme tubulaire de la partie jambière est obtenue par jointure de deux bords (211) de la coque souple.

4. Orthèse de jambe selon la revendication 3, comprenant en outre un système d'attache (3) apte à maintenir les deux bords sensiblement droits joints.

5. Orthèse de jambe selon la revendication 4, dans laquelle le système d'attache comprend une attache (31) prévue sur la partie jambière.

6. Orthèse de jambe selon l'une des revendications 1 à 5, dans laquelle le système d'attache comprend une attache (32) prévue sur la jonction (24) entre la partie jambière et la partie pédieuse.

7. Orthèse de jambe selon l'une des revendications 1 à 6, dans laquelle la partie jambière présente des orifices de ventilation (23).

8. Orthèse de jambe selon l'une des revendications 1 à 7, dans laquelle la partie pédieuse présente une partie talon (223) avec un orifice d'évacuation (2231).

9. Orthèse de jambe selon l'une des revendications 1 à 8, dans laquelle la partie pédieuse comprend une partie plantaire (221) de soutien du pied.

10. Orthèse de jambe selon l'une des revendications 1 à 9, dans laquelle la partie pédieuse comprend une ouverture avant de dégagement des orteils.

11. Orthèse de jambe selon l'une des revendications 1 à 10, comprenant en outre un raidisseur (4) pour l'arrière pied.

## Patentansprüche

1. Beinorthese (1) gegen Spastik, umfassend eine weiche und elastische Schale (2) mit einem Beinteil (21) und einem Fußteil (22), das sich von dem Beinteil erstreckt, **dadurch gekennzeichnet, dass** das Beinteil eine Röhrenform aufweist und das Beinteil und das Fußteil in einem einzigen Stück aus einem einzigen Materialblock hergestellt sind, wobei das einzige Stück aus einem Material mit einer Elastizität von 15 bis 25 N/mm² hergestellt ist.

2. Beinorthese nach Anspruch 1, wobei das einzige Stück aus einem Material mit einer Härte von 65 bis 100 Shore A besteht.

3. Beinorthese nach Anspruch 1 oder Anspruch 2, wobei die Röhrenform des Beinteils durch Verbinden zweier Ränder (211) der weichen Schale erhalten ist.

4. Beinorthese nach Anspruch 3, welche ferner ein Befestigungssystem (3) umfasst, das in der Lage ist, die beiden Ränder im Wesentlichen gerade verbunden zu halten.

5. Beinorthese nach Anspruch 4, wobei das Befestigungssystem eine an dem Beinteil vorgesehene Befestigung (31) umfasst.

6. Beinorthese nach einem der Ansprüche 1 bis 5, wobei das Befestigungssystem ein Befestigungselement (32) umfasst, das an der Verbindungsstelle (24) zwischen dem Beinteil und dem Fußteil vorgesehen ist.

7. Beinorthese nach einem der Ansprüche 1 bis 6, wobei das Beinteil Belüftungslöcher (23) aufweist.

8. Beinorthese nach einem der Ansprüche 1 bis 7, wobei das Fußteil ein Fersenteil (223) mit einer Austrittsöffnung (2231) aufweist.

9. Beinorthese nach einem der Ansprüche 1 bis 8, wobei das Fußteil ein Plantarteil (221) zur Abstützung des Fußes aufweist.

10. Beinorthese nach einem der Ansprüche 1 bis 9, wobei das Fußteil eine vordere Öffnung zur Freigabe der Zehen aufweist.

11. Beinorthese nach einem der Ansprüche 1 bis 10, welche ferner eine Versteifung (4) für den hinteren Fußbereich umfasst.

## Claims

1. Anti-spasticity leg brace (1) comprising a flexible and elastic shell (2) with a leg section (21) and a foot section (22) extending from the leg section, **characterised in that** the leg section has a tubular shape and the leg section and the foot section are made of a single piece made from a single material block, wherein the single piece is a material of 15 to 25 N/mm² elasticity.

2. Leg brace according to claim 1, wherein the single piece is a material of 65 to 100 Shore A hardness.

3. Leg brace according to claim 1 or claim 2, wherein the tubular shape of the leg section is obtained by joining two edges (211) of the flexible shell.

4. Leg brace according to claim 3, further comprising a fastening system (3) capable of keeping the two substantially straight edges joined.

5. Leg brace according to claim 4, wherein the fastening system comprises a fastener (31) provided on the leg section.

6. Leg brace according to one of claims 1 to 5, wherein the fastening system comprises a fastener (32) provided on the junction (24) between the leg section and the foot section.

7. Leg brace according to one of claims 1 to 6, wherein the leg section has ventilation orifices (23).

8. Leg brace according to one of claims 1 to 7, wherein the foot section has a heel section (223) with an evacuation orifice (2231).

9. Leg brace according to one of claims 1 to 8, wherein the foot section comprises a plantar foot support section (221).

10. Leg brace according to one of claims 1 to 9, wherein the foot section comprises a front opening for freeing the toes.

11. Leg brace according to one of claims 1 to 10, further comprising a stiffener (4) for the hindfoot.
